# EUROPÄISCHE PATENTANMELDUNG

(11) **EP 0 760 483 A2**
(43) Veröffentlichungstag der Anmeldung: **05.03.1997**
(21) Anmeldenummer: 96113710.6
(22) Anmeldetag: 27.08.1996
(51) Int. Cl.: G01N 33/68

(54) **Reagenzienkit zur quantitativen Bestimmung von Proteinen und Peptiden**

(30) Priorität: 28.08.1995 DE 29513801 U; 07.09.1995 DE 29514396 U
(71) Anmelder: BOEHRINGER MANNHEIM GMBH, D-68305 Mannheim-Waldhof (DE)
(72) Erfinder: Strobel, Oliver, Dr., 83671 Benediktbeuren (DE); Strobel, Edith, 83671 Benediktbeuren (DE); Von der Eltz, Herbert, Dr., 82362 Weilheim (DE)
(74) Vertreter: Böhm, Brigitte, Dipl.-Chem. Dr.

(57) **Zusammenfassung**

Ein erfindungsgemäßer Reagenzienkit zur quantitativen Bestimmung von Proteinen oder/und Peptiden umfaßt ein Reagenz A, enthaltend 0,7 bis 2 mmol/l Cu²⁺-Ionen und 2 bis 4 mmol/l Tartrat in alkalischer Lösung und ein Reagenz B, enthaltend 1 bis 1,5 mmol/l Ascorbinsäure und 0,5 bis 0,8 mmol/l Bathocuproin, wobei das Volumenverhältnis von Reagenz A zu Reagenz B 1:8 bis 1:12 beträgt und das gemeinsame Volumen von Reagenz A und Reagenz B zwischen 750 µl und 3000 µl liegt.

## Beschreibung

Die Erfindung betrifft einen Reagenzienkit zur quantitativen Bestimmung von Proteinen oder/und Peptiden.

Proteine stellen eine große und wichtige Gruppe der Biomoleküle dar. Aufgrund ihrer weiten Verbreitung und zentralen Rolle in der Natur wurden zahlreiche Methoden zur Messung von Proteinkonzentrationen entwickelt. Die gegenwärtig am häufigsten verwendeten spektrophotometrischen Methoden lassen sich anhand der Verfahren, mit denen eine farbige und somit detektierbare Lösung erzielt wird, in zwei Gruppen einteilen.

Kupfer-Chelat-Bildner-Assays nutzen eine Kupfer-Protein-Komplexbildung unter stark basischen Bedingungen, die sogenannte Biuret-Reaktion zur Detektion.

Farbstoff-Protein-Komplexbildung wird in einer zweiten Gruppe zur Detektion herangezogen, wobei die Farbstoff-Protein-Komplexbildung eine Verschiebung des Absorptionsmaximums des Farbstoffs bewirkt.

Bei der Methode nach Lowry et al. (Lowry, O.H., Rosebrough, N.J., Farr, A.L., and Randall, R.J. (1951) J. Biol. Chem. 193, 265-275) wird das Folin-Ciocalteau-Reagenz eingesetzt, um die Empfindlichkeit der Biuret-Reaktion zu erhöhen. Dadurch wird eine relativ hohe Empfindlichkeit von 0,2 bis 1,4 mg/ml erreicht. Weiterhin zeigt diese Methode eine relativ gute Stabilität und Reproduzierbarkeit gegenüber unterschiedlichen Proteinen. Ein wesentlicher Nachteil dieser Methode ist jedoch die Instabilität des Detektionsreagenzes unter alkalischen Bedingungen.

Die Methode nach Smith et al., (Smith, P.K., Krohn, R.I., Hermanson, G.T., Mallia, A.K., Gartner, F.H., Provenzano, M.D., Fujimoto, E.K. Goeke, N.M., Olson, B.J. and Klenk. D.C. (1985) Anal. Biochem. 150, 76-85) nutzt ebenfalls die Biuret-Reaktion zur Bestimmung von Proteinen. Als Kupfer-Chelat-Bildner wird Bicinchoninsäure (BCA) eingesetzt, die zusammen mit Kupferionen einen detektierbaren lila-farbigen Komplex ergibt. Dieser Assay zeichnet sich durch hohe Sensitivität (0,1 bis 1 bis 2 g/ml) und gute Stabilität der Reagenzien aus. Es treten jedoch hohe Protein-zu-Protein-Schwankungen bei unterschiedlichen Proteinen auf.

Aus der Gruppe der Farbstoff-Protein-Komplex-Assays ist der Bradford Protein-Assay am weitesten verbreitet (Bradford, M.M. (1976) Anal. Biochem. 72, 248-254). Bei diesem Assay wird Coomasie-Brilliant Blau 250 als komplexbildender Farbstoff verwendet. Auch dieser Assay zeigt eine relativ hohe Sensitivität von 0,2 bis 1,4 mg/ml. Nachteile dieses Assays sind hohe Protein-zu-Protein-Schwankungen bei unterschiedlichen Proteinen, sowie die Anfälligkeit des Assays gegenüber Detergentien und alkalischen Puffern.

Ein weiterer Kupfer-Chelat-Protein-Assay wurde von Matsushita et al., beschrieben (Matsushita, M., Irino, T., Komoda, T., and Sakagishi, Y. (1993) Clin. Chim. Acta 216, 103-111). Auch dieses Verfahren nutzt die Biuret-Reaktion zur Bestimmung von Proteinkonzentrationen aus. Zur Detektion wird jedoch überschüssiges Cu⁺⁺ zu Cu⁺ reduziert und der farbige Bathocuproin-Cu⁺-Komplex bestimmt. Diese Methode weist eine niedrige Protein-zu-Protein-Variabilität und eine hohe Stabilität der Reagenzien auf. Nachteilig ist jedoch die Empfindlichkeitsgrenze von 0,4 mg/ml. Weiterhin weist diese Methode eine Reihe von Nachteilen auf, die eine Durchführung als Standard-Protein-Assay erschweren. So werden die Inkubationen bei 37°C durchgeführt, was die Handhabung erschwert und das Gesamtvolumen der Methode ist nicht zur Durchführung des Proteinassays in Standard-Semi-Mikroküvetten geeignet.

Aufgabe der vorliegenden Erfindung war es daher, einen Reagenzienkit zur Bestimmung von Proteinen bereitzustellen, bei dem die Nachteile des Standes der Technik zumindest teilweise beseitigt sind.

Die Aufgabe wird erfindungsgemäß gelöst durch die Bereitstellung eines Reagenzienkits zur quantitativen Bestimmung von Proteinen oder Peptiden, umfassend ein Reagenz A, enthaltend Cu²⁺-Ionen und Tartrat in alkalischer Lösung und ein Reagenz B, enthaltend Ascorbinsäure und Bathocuproin, wobei das Volumenverhältnis von Reagenz A zu Reagenz B 1:8 bis 1:12 beträgt und das gemeinsame Volumen von Reagenz A und Reagenz B zwischen 750 µl und 3000 µl liegt.

Insbesondere wird die Aufgabe gelöst durch die Bereitstellung eines Reagenzienkits zur quantitativen Bestimmung von Proteinen oder Peptiden, umfassend ein Reagenz A, enthaltend 0,7 bis 2 mmol/l Cu⁺⁺-Ionen und 2 bis 4 mmol/l Tartrat in alkalischer Lösung und ein Reagenz B, enthaltend 1 bis 1,5 mmol/l Ascorbinsäure und 0,5 bis 0,8 mmol/l Bathocuproin, wobei das Volumenverhältnis von Reagenz A zu Reagenz B 1:8 bis 1:12 beträgt und das gemeinsame Volumen von Reagenz A und Reagenz B zwischen 750 µl und 3000 µl liegt.

Der erfindungsgemäße Reagenzienkit ermöglicht die spektrophotometrische Bestimmung von Protein- und Peptidkonzentrationen. Durch Zugabe von Reagenz A zu einer Protein enthaltenden Probelösung wird zunächst ein Kupfer-Protein-Komplex entsprechend der Biuret-Reaktion gebildet. Nach einer Inkubationszeit wird Reagenz B zugegeben. Überschüssige Cu⁺⁺-Ionen werden durch ein Reduktionsmittel, wie z.B. Ascorbinsäure zu Cu⁺-Ionen reduziert. Die Cu⁺-Ionen bilden mit einem ebenfalls in Reagenz B enthaltenen Komplexbildner, wie z.B. Bathocuproin einen farbigen Komplex, dessen Absorption gemessen werden kann. Das so erhaltene Signal ist invers proportional zu der Menge an Peptidbindungen und im Gegensatz zu anderen herkömmlichen Protein-Assays unabhängig von den Seitengruppen der Aminosäuren.

Der vorliegende Reagenzienkit ermöglicht eine exakte und sensitive Messung von Protein- und Peptidkonzentrationen. Aufgrund der geringen Protein-zu-Protein-Variabilität können auch Proteingemische mit hoher Genauigkeit gemessen werden. Weitere Vorteile der Proteinbestimmung mit dem vorliegenden Reagenzienkit sind die kurze Assayzeit, gute Reagenzstabilität, hervorragende Sensitivität, breiter linearer Bereich und Toleranz gegenüber möglicherweise störenden Substanzen.

Bevorzugt enthält der Reagenzienkit in Reagenz A Kupfersulfat und/oder Natriumkaliumtartrat. Cu⁺⁺-Ionen und Tartrat können jedoch auch in anderen, dem Fachmann bekannten Formen, eingesetzt werden. Das Reagenz B enthält ein Reduktionsmittel, vorzugsweise Ascorbinsäure und einen Komplexbildner, vorzugsweise Bathocuproindisulfonsäuredinatriumsalz. Grundsätzlich sind jedoch alle Komplexbildner geeignet, die unter alkalischen Bedingungen mit Kupfer einen Komplex bilden.

Die Cu⁺⁺-Ionen liegen in Reagenz A bevorzugt in einer Konzentration von 0,8 bis 1,2 mmol/l und besonders bevorzugt von 0,86 mmol/l vor. Reagenz A enthält außerdem Tartrat in einer bevorzugten Konzentration von 2 bis 2,5 mmol/l und besonders bevorzugt von 2,28 mmol/l. Zudem enthält Reagenz A bevorzugt eine Base, beispielsweise NaOH in einer Konzentration von 700 bis 1000 mmol/l.

Reagenz B enthält ein Reduktionsmittel vorzugsweise in einer Konzentration 1,3 bis 1,35 mmol/l und besonders bevorzugt von 1,32 mmol/l. Der Komplexbildner liegt in Reagenz B bevorzugt in einer Konzentration von 0,6 bis 0,64 mmol/l und besonders bevorzugt von 0,62 mmol/l vor.

Um eine einfache Handhabung unter Verwendung von üblichen Laborgeräten zu ermöglich ist die Volumenmenge des Protein-Assays so ausgelegt, daß eine Durchführung in Standard-Semimikroküvetten oder Standard-Mikroküvetten möglich ist. Bevorzugt ist die Ausführung in Standard-Semimikroküvetten, wobei das Gesamtvolumen der Reagenzien 900 µl bis 1500 µl beträgt.

Der erfindungsgemäße Reagenzienkit enthält außerdem bevorzugt eine Pufferlösung, mit der Standard- und Probenlösungen vorbereitet werden können. Zusätzlich enthält der Reagenzienkit A bevorzugt eine Protein-Standardlösung. Die Protein-Standardlösung liegt vorzugsweise in einer Konzentration von 20 µg/ml bis 2 mg/ml vor. Der Reagenzienkit enthält bevorzugt eine höher konzentrierte Protein-Stammlösung, aus der geeignete Standardlösungen durch Verdünnen hergestellt werden. Besonders bevorzugt ist eine Protein-Stammlösung, die Rinderserumalbumin mit einer Konzentration von 2 mg/ml enthält. Die geeignet verdünnte Protein-Standardlösung wird bevorzugt in einem Volumenverhältnis von 1:1,6 bis 1:2,4, bezogen auf Reagenz A für einen Assay eingesetzt.

Der vorliegende Reagenzienkit und das zugehörige Verfahren zeichnen sich vor allem durch eine einfache Handhabbarkeit, eine leichte Durchführbarkeit der quantitativen Bestimmung von Proteinen und Peptiden sowie eine geringe Protein-zu-Protein-Variabilität und ein niedriges Detektionslimit aus.

Das dem Assay zugrundeliegende und als solches bekannte Verfahren umfaßt drei Schritte. Im ersten Schritt wird protein- oder peptidhaltige Probelösung mit alkalischer Cu⁺⁺-Tartratlösung umgesetzt, wobei sich ein Cu⁺⁺-Protein-Komplex bildet. Im zweiten Schritt wird überschüssiges Cu⁺⁺ mit einem Reduktionsmittel, insbesondere Ascorbinsäure zu Cu⁺ reduziert. Dieses Cu⁺ bildet anschließend mit einem Komplexbildner, z.B. Bathocuproin oder ein anderer alkalistabiler Komplexbildner einen farbigen Komplex der zur quantitativen Detektion herangezogen wird. Für die Durchführung des Assays enthält Reagenz A die Reagenzien für den ersten Schritt (entspricht Reaktion 1), während Reagenz B die Reagenzien für den zweiten und dritten Schritt umfaßt (entspricht Reaktion 2). Die erfindungsgemäße Volumenmenge des Assays beträgt 750 bis 3000 ml, wobei ein Probevolumen von 15 bis 150 µl eingesetzt wird.

Das Gesamtvolumen eines Assays ist so ausgelegt, daß die Durchführung in Standardküvetten möglich ist. Dadurch kann der Assay mit laborüblichen Materialien ausgeführt werden ohne die Notwendigkeit von teuren Spezialgeräten. Bevorzugt wird der Assay in Standard-Semimikroküvetten durchgeführt, wobei die Nachweisreaktionen vollständig in einer Küvette erfolgen können. Das Assay-Verfahren beträgt dabei bevorzugt 900 bis 1500 µl.

Der Assay kann bei 37°C durchgeführt werden, was allerdings eine Temperierung des Ansatzes nötig macht. Bevorzugt wird der Assay deshalb bei Raumtemperatur ohne wärmeregulierende Maßnahmen durchgeführt, was eine weitere wesentliche Vereinfachung der Handhabbarkeit darstellt.

Die Ausbildung der detektierbaren Farbe in Reaktion 2 ist abhängig von der Inkubationszeit der Reaktion 2. Um reproduzierbare und vergleichbare Ergebnisse zu erzielen, muß die Absorption nach einer bestimmten Inkubationszeit der Reaktion 2 gemessen werden. Überraschenderweise wurde festgestellt, daß unter Verwendung der erfindungsgemäßen Konzentrationen und Volumina eine Veränderung der Inkubationszeit der Reaktion 1 von 5 bis 70 Minuten die Meßergebnisse nicht verändert. Dadurch wird eine weitere wesentliche Vereinfachung der Handhabbarkeit erreicht, da bei zeitlicher Unabhängigkeit der Reaktion 1 die zeitlich abhängige Reaktion 2 zu einem dem Benutzer geeigneten Zeitpunkt gestartet werden kann. Weiterhin kann dadurch eine Probe in nur 7 Minuten vorbereitet und gemessen werden.

Durch die Verwendung der erfindungsgemäßen Konzentrationen zeichnet sich der Assay durch ein sehr niedriges Detektionslimit aus. So können Konzentrationen von bis zu 20 µg Protein bzw. Peptid pro ml Probelösung gemessen werden. Der lineare Bereich ist abhängig von den Reagenzienkonzentrationen. Durch Veränderung der Reagenzienkonzentration wird der lineare Bereich aber auch das Detektionslimit verändert. Je nach Aufgabenstellung wird somit eine Optimierung des Detektionslimits oder ein möglichst großer linearer Bereich durch Verwendung der geeigneten Konzentrationen erhalten.

Das in einem Assay eingesetzte Probevolumen beträgt vorzugsweise 15 bis 150 µl. Zusammen mit dem niedrigen Detektionslimit ermöglicht dies die direkte Verwendung vieler protein- bzw. peptidhalter Probeflüssigkeiten ohne die Notwendigkeit vorheriger Aufkonzentrierungsschritte. Insbesondere müssen z.B. Protein- bzw. Peptidlösungen, die über Säulen gereinigt wurden, vor Bestimmung der Proteinkonzentration nicht von überschüssigem Lösungsmittel befreit werden.

Weiterhin können mit dem vorliegenden Assay verschiedene Proteine ohne große Schwankungen gemessen werden. Eine geringe Protein-zu-Protein-Variabilität ist vor allem bei der Messung von unterschiedlichen Proteinen oder von Proteingemischen von Bedeutung. Der vorliegende Proteinassay zeigt eine geringe Protein-zu-Protein-Variabilität, wodurch verschiedene Proteine exakt quantitativ bestimmt werden können.

Der Protein-Assay zeichnet sich weiterhin durch hervorragende Reagenzienkompatibilität aus, so daß eine Störung durch Verunreinigungen der Probe, Puffer oder Hilfsmittel weitgehend ausgeschlossen werden kann.

Da mit dem vorliegenden Reagenzienkit neben Proteinen auch Peptide quantitativ bestimmt werden können, ergibt sich eine weite Anwendbarkeit.

Zur Automatisierung kann der Assay auch auf Mikrotiterplatten durchgeführt werden, wobei das Volumen für eine Küvette auf 10 Wells aufgeteilt wird. Daneben ist der vorliegende Protein-Assay auch geeignet zur quantitativen Bestimmung von immobilisierten Proteinen, wie z.B. solchen, die an eine feste Matrix, wie z.B. Agarose gebunden sind.

Die Erfindung wird durch die folgenden Beispiele näher erläutert.

### Beispiel 1

### Standard-Assay-Verfahren / Erstellen einer Eichkurve

Reagenz A, umfassend 0,86 mM CuSO₄ x 5H₂O, 2,28 mM Natrium Kalium Tartrat und 0,86 NaOH und Reagenz B, umfassend 1,32 mM Ascorbinsäure und 0,62 mM Bathocuproin-Disulfonsäure-Dinatriumsalz werden auf Raumtemperatur oder 37°C erwärmt. Eine Reihe Standardlösungen, die den zu messenden Konzentrationsbereich überdeckt, wird durch Verdünnen der im Reagenzienkit enthaltenen BSA-Proteinstandardlösung auf 0, 50, 100, 300, 500 und 750, 1000, 1500 und 2000 µg/ml hergestellt.

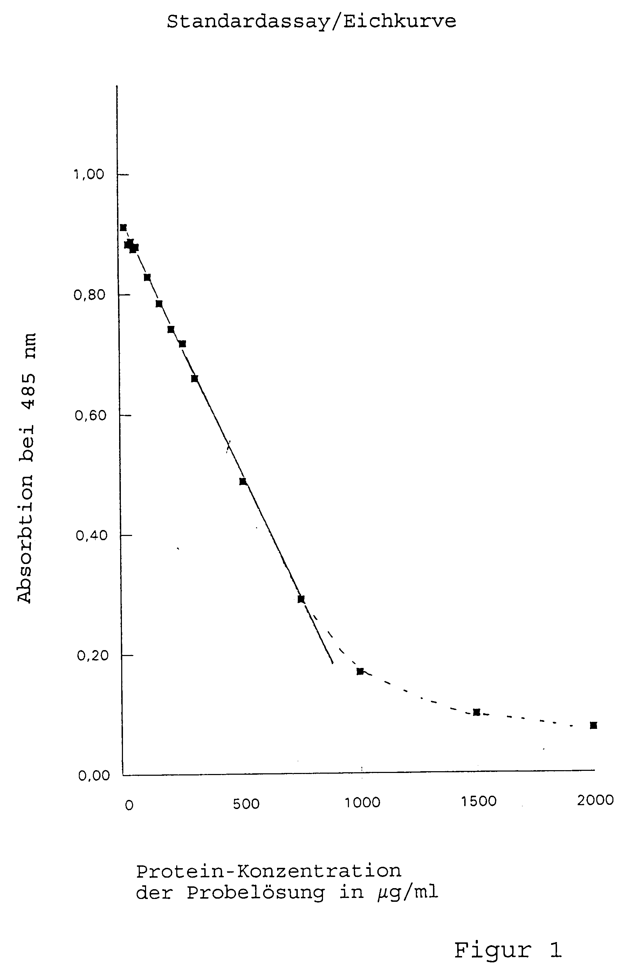

In eine Semi-Mikroküvette (1,5 ml) werden 100 µl Reagenz A zugegeben. Anschließend werden 50 µl Standardlösung bzw. Probelösung zu der Küvette zugegeben und mit Reagenz A vermischt. Die Lösungen werden bei Raumtemperatur für mindestens 5 Minuten inkubiert. Längere Inkubationszeiten von bis zu 1 Stunde beeinflußen das Ergebnis des Protein-Assays nicht. Anschließend werden 1000 µl Reagenz B zu der Küvette zugegeben und kurz vermischt. Nach 30 Sekunden wird die Absorption bei 485 nm gemessen. Eine mit BSA erhaltene Eichkurve ist in Figur 1 gezeigt. Der Assay weist einen linearen Bereich von 20 bis 800 µg Protein pro ml Probenlösung auf (Absolut: 1 bis 40 µg Protein pro Assay). Anhand einer solchen Eichkurve kann die Proteinkonzentration von unbekannten Proben berechnet werden. Bei wiederholten Messungen von Proben mit der gleichen Matrix genügt es normalerweise, einmalig eine Eichkurve zu erstellen und die Gültigkeit dieser Eichkurve dann nur noch an einem Punkt, z.B. am Nullpunkt zu überprüfen.

### Beispiel 2

### Linearer Bereich / Änderung der Reagenzienkonzentrationen

Ein Protein-Assay wird analog Beispiel 1 durchgeführt, wobei das Reagenz A 1,1 mmol/l Kupfersulfat und 2,9 mmol/l Kaliumnatriumtartrat enthält. Der lineare Bereich des Assays konnte dadurch auf den Bereich von 100 bis 1500 µg Protein pro ml Probenlösung erweitert werden.

### Beispiel 3

### Linearer Bereich / Änderung der Reagenzienkonzentrationen

Ein Protein-Assay wird analog Beispiel 1 durchgeführt, wobei das Reagenz A 1,3 mmol/l Kupfersulfat und 3,4 mmol/l Kaliumnatriumtartrat enthält. Der lineare Bereich des Assays konnte dadurch auf den Bereich von 100 bis 1700 µg Protein pro ml Probenlösung erweitert werden.

### Beispiel 4

### Inkubationszeit der ersten Reaktion

Der Protein-Assay wurde analog Beispiel 1 ausgeführt, wobei die erste Reaktion für 5, 20 und 70 Minuten bei Raumtemperatur durchgeführt wurde. Eine Veränderung der Ergebnisse aufgrund der unterschiedlichen Inkubationszeiten der ersten Reaktion wurde nicht beobachtet.

### Beispiel 5

### Temperaturabhängigkeit

Der Protein-Assay wurde analog Beispiel 1 bei Raumtemperatur und bei 37°C ausgeführt. Es wurde keine Veränderung des linearen Bereichs und des unteren Detektionslimits bei den verschiedenen Temperaturen festgestellt. Durch die Durchführung bei Raumtemperatur wird eine wesentliche Vereinfachung der Handhabbarkeit erzielt.

### Beispiel 6

### Protein-zu-Protein-Variabilität

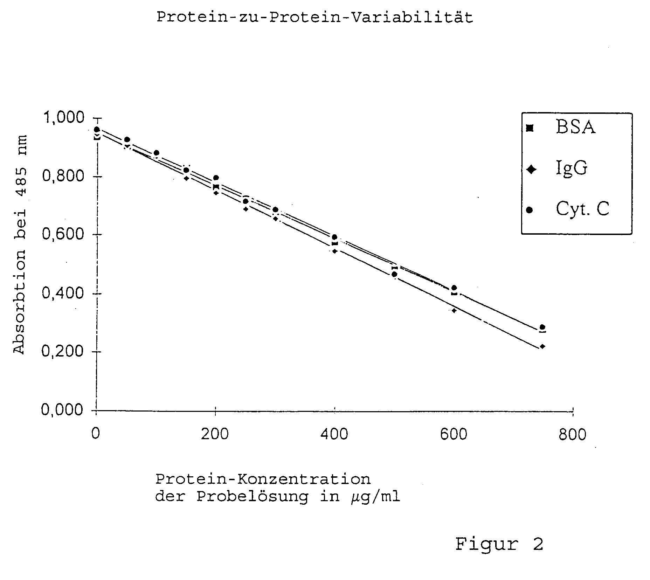

Der Assay wurde auf Proben von Maus-Immunglobulin-G, Pferde-Cytochrom C und Rinderserumalbumin getestet. Die in Figur 2 gezeigten Ergebnisse belegen, daß eine sehr geringe Variation bei unterschiedlichen Proteinen auftritt.

### Beispiel 7

### Reagenzienkompatibilität

Der Assay wurde analog Beispiel 1 durchgeführt. Die in der folgenden Tabelle aufgeführten Verbindungen wurden zu den Protein-Standardlösungen zugegeben. Eine Veränderung der Meßergebnisse wurde nicht beobachtet, was die Unempfindlichkeit des Verfahrens gegenüber Puffern und anderen Zusatzstoffen veranschaulicht.

| | | |
|---|---|---|
| 1 % Octylglucosidase (w/v) | 0,5 M Hepes, pH 5,1 | 0,5 M Ammoniumsulfat |
| 0,5 % Triton® X-100 (w/v) | 0,5 M Natriumphosphat, pH 7,5 | 0,5 M Ammoniumacetat |
| 0,5 % Brij® 35 (w/v) | 0,1 M Glycin, pH 2,8 | 0,1 % Micr-O-Protect (v/v) |
| 0,5 % Thesit® (w/v) | | 5 mM CaCl₂ |
| 0,5 % Nonidet® P-40 (w/v) | | 0,2 mM DTT |
| 0,5 % CHAPS (w/v) | | 0,1 % Natriumazid (w/v) |

## Patentansprüche

1. Reagenzienkit zur quantitativen Bestimmung von Proteinen oder/und Peptiden, umfassend ein Reagenz A, enthaltend 0,7 bis 2 mmol/l Cu²⁺-Ionen und 2 bis 4 mmol/l Tartrat in alkalischer Lösung und ein Reagenz B, enthaltend 1 bis 1,5 mmol/l Ascorbinsäure und 0,5 bis 0,8 mmol/l Bathocuproin, wobei das Volumenverhältnis von Reagenz A zu Reagenz B 1:8 bis 1:12 beträgt und das gemeinsame Volumen von Reagenz A und Reagenz B zwischen 750 µl und 3000 µl liegt.

2. Reagenzienkit nach Anspruch 1,
**dadurch gekennzeichnet,**
daß Reagenz A CuSO₄ oder/und Natriumkaliumtartrat enthält.

3. Reagenzienkit nach einem der Ansprüche 1 oder 2,
**dadurch gekennzeichnet,**
daß Reagenz B Bathocuproindisulfonsäuredinatriumsalz enthält.

4. Reagenzienkit nach Anspruch 1,
**dadurch gekennzeichnet,**
daß Reagenz A Cu²⁺ in einer Konzentration von 0,8 bis 1,2 mmol/l oder/und Tartrat in einer Konzentration von 2 bis 2,5 mmol/l enthält.

5. Reagenzienkit nach einem der Ansprüche 1 bis 4,
**dadurch gekennzeichnet,**
daß Reagenz B Ascorbinsäure in einer Konzentration von 1,3 bis 1,35 mmol/l oder/und Bathocuproin in einer Konzentration von 0,6 bis 0,64 mmol/l enthält.

6. Reagenzienkit nach Anspruch 1 bis 5,
**dadurch gekennzeichnet,**
daß das Gesamtvolumen der Reagenzien zwischen 900 µl und 1500 µl beträgt.

7. Reagenzienkit nach einem der Ansprüche 1 bis 6,
**dadurch gekennzeichnet,**
daß eine Pufferlösung enthalten ist.

8. Reagenzienkit nach einem der Ansprüche 1 bis 7,
**dadurch gekennzeichnet,**
daß eine Protein-Standardlösung enthalten ist.

9. Reagenzienkit nach einem der Ansprüche 1 bis 8,
**dadurch gekennzeichnet,**
daß die Konzentration der Protein-Standardlösung 20 µg/ml bis 2 mg/ml beträgt.

10. Reagenzienkit nach einem der Ansprüche 1 bis 9,
**dadurch gekennzeichnet,**
daß das Volumenverhältnis von Protein-Standardlösung zu Reagenz A für einen Assay 1:1,6 bis 1:2,4 beträgt.

11. Reagenzienkit nach einem der Ansprüche 1 bis 10,
**dadurch gekennzeichnet,**
daß als Proteinstandardlösung eine Rinderserumalbuminlösung enthalten ist.
